# EUROPEAN PATENT SPECIFICATION

(11) **EP 2 142 253 B1**
(45) Date of publication and mention of the grant of the patent: **07.03.2018**
(21) Application number: 08769197.8
(22) Date of filing: 26.04.2008
(51) Int. Cl.: A61N 1/372, G06Q 50/00

(54) **SYSTEM FACILITATING AUXILIARY ANALYSIS OF DATA FROM IMPLANTED MEDICAL DEVICES**
SYSTEM ZUM ERLEICHTERN DER ANALYSE VON DATEN AUS IMPLANTIERTEN MEDIZINISCHEN VORRICHTUNGEN
SYSTÈME FACILITANT L'ANALYSE SECONDAIRE DE DONNÉES ISSUES DE DISPOSITIFS MÉDICAUX IMPLANTÉS

(30) Priority: 27.04.2007 US 741031
(43) Date of publication of application: 13.01.2010
(73) Proprietor: MEDTRONIC, INC., Minneapolis, Minnesota 55432 (US)
(72) Inventor: PATEL, Amisha S., Apex, North Carolina 27502 (US); WEBB, James D., Maple Grove, Minnesota 55311 (US)
(74) Representative: Hughes, Andrea Michelle
(86) International application number: PCT/US2008/061694
(87) International publication number: WO 2008/134598

(56) References cited:
- WO-A-2006/119340
- US-A1- 2004 015 197
- US-A1- 2004 120 557
- US-A1- 2005 192 649
- US-A1- 2008 065 165
- US-B1- 6 270 457
- US-B2- 6 842 645
- US-B2- 7 047 083

## Description

### TECHNICAL FIELD

The present invention relates to implantable medical devices and more particularly to systems for facilitating auxiliary analyses of data collected by the implanted devices.

### BACKGROUND

In recent years the capacity of various types of implantable medical devices to collect and store large amounts of data has dramatically increased. These devices include microprocessors for processing collected data via one or more pre-programmed algorithms. The one or more algorithms detect and classify sensed episodes, which are characteristic of certain patient conditions, for example, cardiac arrhythmias, and which could benefit from therapy delivery, thereby guiding therapy delivery from the device. The one or more algorithms may also monitor device integrity, for example, by tracking events characteristic of sensing issues and/or device impedance changes. With respect to the latter, certain components of implantable medical devices, like any other man-made device, are subject to fault or failure, for example, either due to operator error at the time of implant or due to normal "wear and tear" on the components during the life of the device. These faults or failures can result in artifact signals which are sensed by the device and mistaken for physiological signals, thereby impacting the accuracy of the data analysis performed by the device in classifying episodes. Due to constraints such as a time sensitive nature of some conditions, which could be life-threatening, combined with a complexity of analysis required to come to some conclusion, there is a limitation on the amount of analysis that the device microprocessor can undertake in order to provide a comprehensive verification of an accuracy of an initial detection and classification of an episode and/or verification of a device integrity issue. Implantable device size constraints, resulting in power limitations of the device, may alternately or additionally limit the amount of data processing/analysis performed by the device.

Many sophisticated data processing algorithms have been developed to perform more detailed analyses of data collected by implanted medical devices. Some of these algorithms have been programmed on an external medical device that can be wirelessly coupled, for example, via telemetry, to an implanted device for the transfer of the data from the implanted device to the external device. Alternately, some of the algorithms have been programmed on computers, which are not wirelessly coupled to the device, and the data is transferred from external medical devices to the computers, for example personal or lap top computers, on a disk. These algorithms can process the data to come to some conclusions regarding episode classification and events indicative of device integrity issues, and may further provide a presentation of the data in a format that allows a physician or clinician to further analyze the data. Such auxiliary analyses of data transferred from an implanted device can bring to light device integrity issues and/or errors in one or more analyses performed by the implanted device that have led to misclassification of episodes detected by the device, and thus help an attending physician in making decisions to reposition or replace certain portions/components of the device due to faults or failures detected by the auxiliary analysis, and/or in making decisions related to re-programming of the implanted device in order to prevent misclassification errors in the future. Methods employed by an algorithm, for post processing of data associated with arrhythmic episodes, which are detected and classified by an implantable cardioverter defibrillator (ICD), are described in co-pending and commonly assigned U.S. patent application serial number 11/564,120, published as US 2007/0167986, entitled METHOD AND APPARATUS FOR POST-PROCESSING OF EPISODE DETECTED BY A MEDICAL DEVICE. Examples of methods employed by algorithms that are tailored to identify and classify events indicative of a device integrity issue, in particular, faults or failures associated with lead components of the device, are described in co-pending and commonly assigned U.S. patent application serial number 11/536,364, published as US 2008/0082012, entitled TROUBLESHOOTING METHODS FOR A MEDICAL SYSTEM INCLUDING IMPLANTABLE COMPONENTS, and in commonly assigned U.S. patent publications 2004/0015197, 2005/0137636, and in U.S. Patent No. 7,047,083. US 2004/120557, US 6,270,457 and US 6,842,645 disclose systems and techniques for collecting and analyzing medical data.

US 2005/192649 and US 2008/065165 also teach data analysis techniques.

Although the above-referenced methods, as well as many others known to those skilled in the art, are available to assist in auxiliary analyses of data collected from implanted devices, there is a need for systems and methods that provide for a more automated and orderly approach to auxiliary analysis. Such systems and methods can help physicians and clinicians to better manage a growing population of patients in which medical devices are implanted.

The present invention provides a system as defined by claim 1.

### BRIEF DESCRIPTION OF THE DRAWINGS

The following drawings are illustrative of particular embodiments of the present invention and therefore do not limit the scope of the invention. The drawings are not to scale (unless so stated) and are intended for use in conjunction with the explanations in the following detailed description. Embodiments of the present invention will hereinafter be described in conjunction with the appended drawings, wherein like numerals denote like elements.
Figure 1 is a schematic depiction of an exemplary system, according to some embodiments of the present invention.
Figures 2A-B are a first view and a second view, respectively, of an exemplary initial page, or screen, of a user interface, according to some embodiments of the present invention.
Figure 3 is a view of a subsequent page presenting a detailed report, according to some embodiments of the present invention.
Figure 4A is a view of another subsequent page presenting one or more graphical displays, according to some embodiments of the present invention.
Figures 4B-C are examples of some graphical displays which may be presented on the page of Figure 4A.
Figure 5 is an example of an impedance trend plot which may be presented on another subsequent page, according to some embodiments of the present invention.

### DETAILED DESCRIPTION

The following detailed description is exemplary in nature and is not intended to limit the scope, applicability, or configuration of the invention in any way. The scope of the invention is defined by the appended claims. Rather, the following description provides practical illustrations for implementing exemplary embodiments of the present invention. Constructions, materials, dimensions, and manufacturing processes suitable for making embodiments of the present are known to those of skill in the field of the invention.

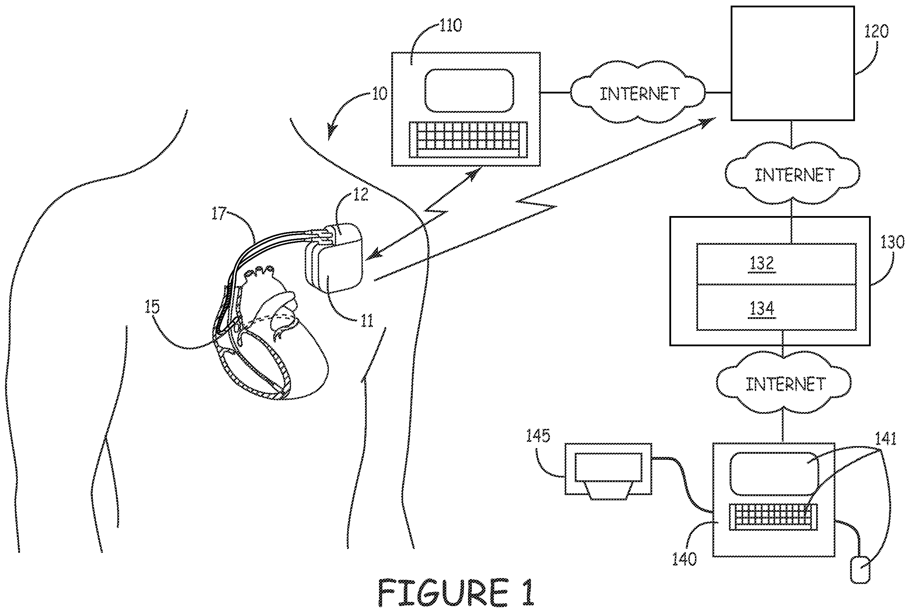

Figure 1 is a schematic depiction of an exemplary system according to some embodiments of the present invention. Figure 1 illustrates an exemplary medical device 10 implanted in a patient; device 10 includes a housing 11, which contains a battery and electronics, a connector module 12, which is coupled to housing 11 and provides electrical connections to the electronics contained within housing 11, and two medical electrical leads 15, 17, which are each coupled to connector module 12. According to an exemplary embodiment of the present invention, device 10 is an ICD wherein lead 15 is implanted in a right atrium, for example, to pace and/or to sense and/or to defibrillate, and lead 17 is implanted in a right ventricle, for example, to pace, sense and defibrillate; those skilled in the art are familiar with details enabling the construction, implantation and function of ICD's. Figure 1 further illustrates an external medical device 110, for example, a programmer or a home monitor for device 10, such as are known to those skilled in the art; external device 110 is wirelessly coupled, for example, via telemetry, to device 10 in order to receive, from device 10, data collected by sensing components, for example, electrodes of leads 15,17, of device 10; the data may be collected in real time, or transferred from a memory of device 10. According to preferred embodiments of the present invention, the collection of data received into programmer from device 10 includes data sets that describe episodes detected and classified by analyses performed by device 10, for example, episodes associated with cardiac arrhythmias, and/or events detected by device 10 to be indicative of a device integrity issue. According to some embodiments of the present invention, external device 110 includes one or more algorithms programmed thereon for performing automated analyses of the data sets. However, according to preferred embodiments of the present invention, external device is linked, for example, via an internet, telecommunications or other type of network connection, to a remote data handling system 120, for transfer of the data sets thereto, so that the data sets are accessible, for example via the internet, for retrieval and analysis by an analysis service 132, for example, a web service, which packages the one or more algorithms that perform the automated analyses of the data sets.

It should be noted that, although exemplary device 10 is shown including particular components, or elements, systems of the present invention may be operational for any type of implantable device that collects data. Furthermore, embodiments of the present invention include systems which are adapted to handle and analyze data from a plurality of medical devices, for example those implanted in a population of patients, which may exceed 100,000 in number; data from each of the plurality of devices may be stored in a database, for example, data handling system 120, and analysis service 132 can identify those patients from the population that require attention.

According to the illustrated embodiment, analysis service 132 is programmed on a server 130, and a software application 134, also programmed on server 130, is adapted to invoke service 132 to retrieve and analyze selected data sets from data handling system 120. Figure 1 shows application 134 linked, for example, via an internet connection, to a personal or lap top computer 140, which supports a user interface 141 projected by application 134; external medical device 110 may also support a user interface projected by application 134. A user of application 134, for example a physician or clinician, may interact with user interface 141 to select data sets and to invoke analysis service 132, so that service 132 retrieves and analyzes the selected data sets. Alternately, or additionally, application 134 is programmed to invoke analysis service 132 at pre-determined time intervals, or at a time when data is being transferred from an implanted device, to retrieve and/or analyze a pre-determined group of data sets, according to pre-programmed criteria. It should be noted that the data sets may encompass data from one or more implanted devices in a single patient and/or from one or more devices implanted in a population of patients, for example, grouped according to association with a particular clinic or geography. If application 134 is programmed to invoke service 132 at pre-determined time intervals, messages informing of the invocation, may be generated by application 134 and sent, for example via an e-mail or a page, to a user of application 134. In any of these instances, application 134 is adapted to notify the user of analysis results and to organize results of the analysis service analysis; the notification and/or organized results may be viewable on user interface 141, and/or on a printout from a printer 145, shown coupled to computer 140, in the form of a report, which may be formatted, for example, into browser screens, into PDF or XPS reports, into e-mail messages, pages, faxes or RSS feeds.

Analysis service 132 may be a type of multi-function data exchange web service described in commonly assigned and co-pending patent application US 2005/0228693 entitled DATA EXCHANGE WEB SERVICES FOR MEDICAL DEVICE SYSTEMS. It should be noted that the configuration of system elements illustrated in Figure 1 is only one type of configuration supporting embodiments of the present invention, and that alternate configurations of elements should be considered to be within the scope of the present invention. For example, although Figure 1 shows analysis service 132 and software application 134 programmed on a server 130 and remotely accessible from any computer via an internet link, the invention is not so limited, and application 134, may be programmed on external medical device 110 and/or computer 141, and may link to analysis service 132 on server 130 via an internet connection; alternately, both service 132 and application 134 may run together locally, for example, on external medical device 110 and/or computer 141. Furthermore, application 134 may be deployed either in a browser or on the desktop. Additionally, although Figure 1 illustrates remote data handling system 120, it should be understood that, according to alternate embodiments, remote data handling system 120 may be replaced by a local database residing on the same machine on which the automated data analysis is performed, and wherein the data is loaded in any manner known to those skilled in the art.

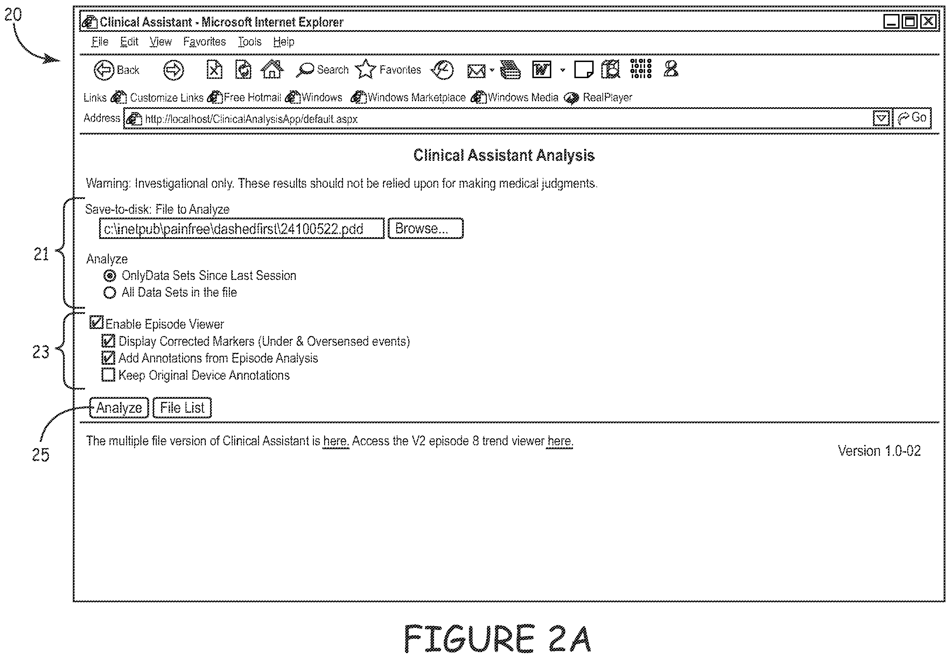

No matter how analysis service 132 and software application 134 are deployed and accessed, embodiments of the present invention provide a tool to perform automated analyses of one or more selected data sets, which have been collected, for example, by device 10, and a plurality of other implanted devices, thereby facilitating an auxiliary analysis of the selected data sets. Figures 2A-4C present an example of user interface 141 projected by software application 134 to enable a user, such as a clinician or physician, to use the tool.

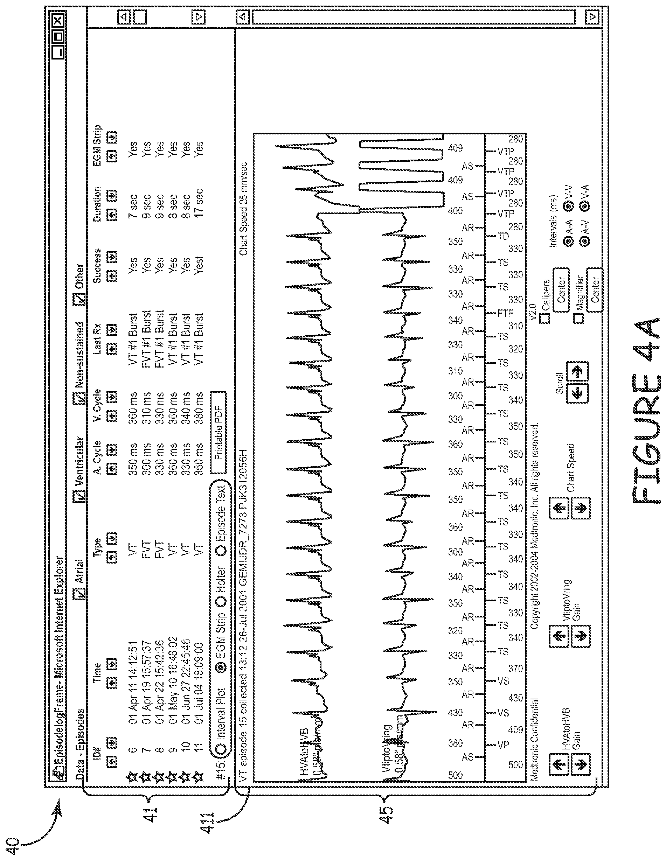

Figure 2A is a view of an initial screen of user interface 141, or a page 20, having an exemplary title of "Clinical Assistant". Figure 2A illustrates initial page 20 displaying an interactive element 21, which the user of the application may utilize to select a group of data sets from a collection of data, either stored on a local database or on a remote database (i.e. remote data handling system 120), that the user would like retrieved and analyzed, for example, by analysis service 132. According to the illustrated example, interactive element 21 allows the user to select a group of data sets by browsing through a listing of save-to-disk files corresponding to memory dumps from device 10; the files may have been translated from a binary to an XML format by analysis service 132. Element 21, as illustrated, also allows the user to choose only those data sets collected since the last session, or interaction with the file, or all data sets collected in the file. Once the data sets are selected, the user may invoke, via an "Analyze" interactive element 25, an automated analysis of each of the selected data sets, for example, performed by analysis service 132. However, Figure 2A further illustrates initial page 20 displaying another interactive element 23, which the user may choose to utilize prior to clicking on element 25, in order to enable an Episode Viewer, which will present a graphical display of selected episodes, for example, including one or more EGM strips and a corresponding marker channel (Figures 4A-B). Element 23, as illustrated, also allows the user, who selects to enable the Episode Viewer, to further select whether or not to correct the display according to the automated analysis results (Episode Analysis), and, if a corrected display is selected, whether to add annotations from the analysis (Annotations from Episode Analysis) or to keep annotations from the device analysis (Original Device Annotations).

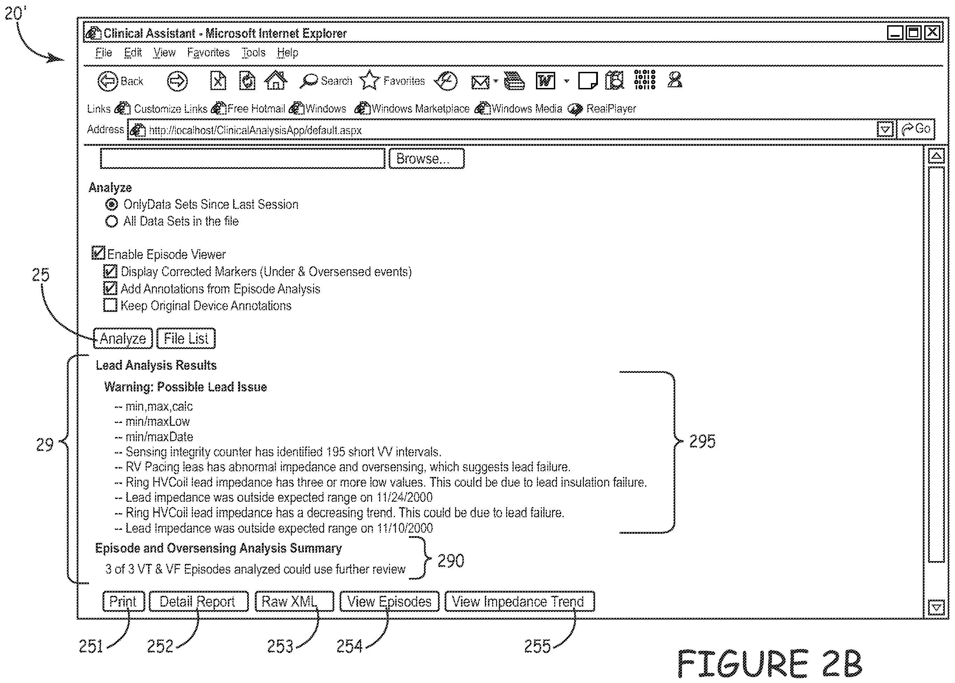

According to the exemplary embodiment, analysis service 132 employs one or more analysis algorithms which are designed to find indications of one or more of the following: oversensing on the part of device 10, undersensing on the part of device 10, abnormal impedance for device 10, supra-ventricular tachyarrhythmia, atrial fibrillation, atrial flutter, atrial/sinus tachyarrhythmia and monomorphic ventricular tachyarrhythmia. Methods which can be incorporated by such an algorithm are described in the aforementioned incorporated references, are known to those skilled in the art, and may employ one or more of a number of well known techniques such as conditional logic, rule based logic, neural nets, bayes nets, etc... Once the automated analysis is complete, page 20 is revised to look like page 20' shown in Figure 2B.

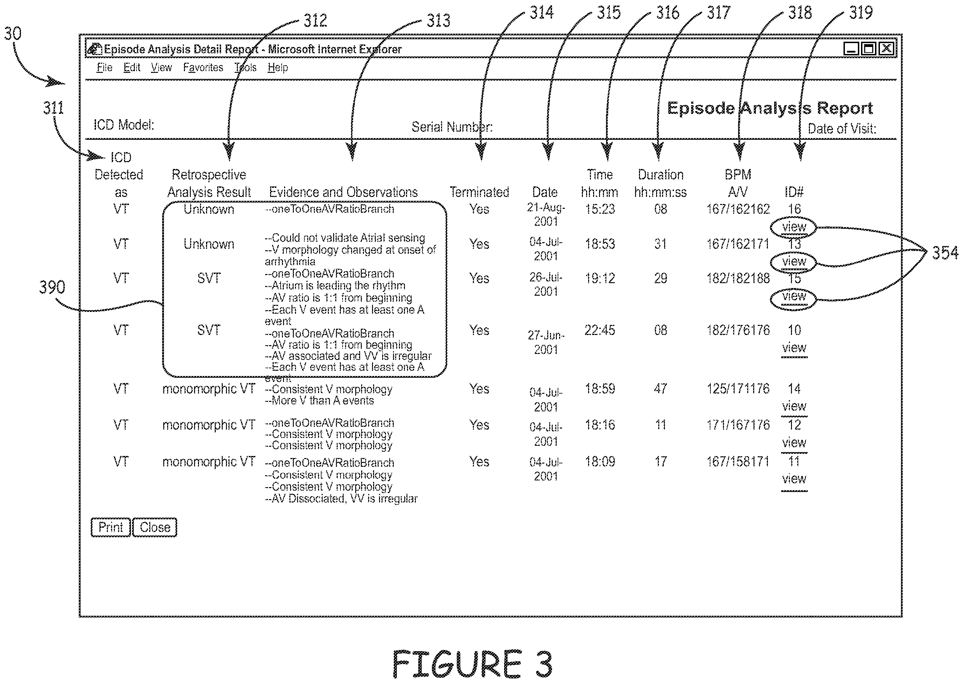

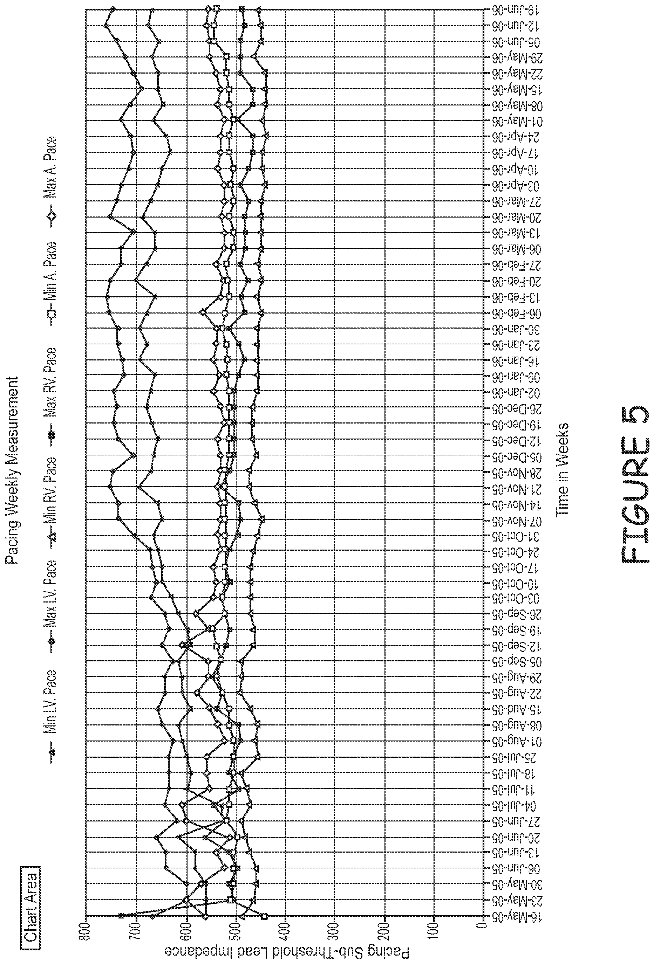

Figure 2B illustrates page 20' displaying a report 29 in the form of a summary of results from the automated analysis; report 29 includes an "Episode and Oversensing Analysis Summary" portion 290 and a "Lead Analysis Results" portion 295. According to the illustrated embodiment, portion 290 provides an indication of a number of episodes, which were detected and classified as Ventricular Tachyarrhythmia (VT) and Ventricular Fibrillation (VF) by device 10, but were classified differently by the automated analysis and/or in which the analysis detected oversensing. Portion 295 of report 29 summarizes evidence, for example, related to oversensing, undersensing, and/or abnormal impedance, which indicates that there may be a device integrity issue in the form of a lead issue, and provides a warning statement to that effect. With reference back to Figure 1, examples of such device integrity issues include, without limitation, a faulty connection between one or both of leads 15, 17 and connector module 12, a conductor fracture and/or insulation breach for one or both of leads 15, 17, and a dislodgement of one or both of leads 15, 17. Figure 2B further illustrates page 20' including four more interactive elements 251, 252, 253, 254, and 255; element 251 to print report 29, element 252 to view a detail report (Figure 3), element 253 to view raw XML data associated with the analysis results, element 254 to view one or more of the analyzed episodes (Figures 4A-C), and element 255 to view a trend plot of impedance (Figure 5).

Figure 3 is a view of a page 30, having an exemplary title of "Episode Analysis Detail Report"; page 30 appears when the user clicks on interactive element 252 of page 20'. Figure 3 illustrates page 30 displaying more detailed information, associated with each episode, organized into nine columns 311-319, and displaying descriptions 390 in a more prominent fashion. Descriptions 390 are displayed in a more prominent fashion by presenting descriptions 390 closer to a top of page 30, than the descriptions of other results, and by bolding the text and highlighting around the text in the "retrospective analysis result" column 312 and in the "evidence and observations supporting the result" column 313. It should be appreciated that the text of one or both of columns 312, 313 for results 390 may alternately, or additionally, be in a different color from the remaining text on page 30 in order to display descriptions 390 more prominently than the rest. Such a display, as is shown in Figure 3, effectively triages episodes for the user and thereby facilitates an auxiliary review of the data on the part of the user, so that time is not wasted reviewing those episodes for which the automated analysis classification essentially matches the device classification.

According to an exemplary embodiment of the present invention, those clinically significant episodes that warrant further review, as determined by the automated retrospective analysis of the analysis service, and are thus displayed in a more prominent fashion on page 30, are further organized, or arranged, according to the following hierarchy: unknown episodes, or those data sets which could not be classified by the retrospective analysis, most prominently displayed, for example, closest to the top of page 30; inappropriately classified episodes, or those episodes that the device determined required therapy but actually did not require therapy, according to the retrospective analysis (-for example, SVT classified as VT/VF by the device), following the unknown; inappropriately treated episodes according to the retrospective analysis (-for example, an episode that could have been pace-terminated rather than shock-terminated), following the inappropriately classified; and other noteworthy episodes, for which the automated retrospective analysis results can provide further insight (-for example, side-effects of appropriate therapy, like accelerated rhythm), following the inappropriately treated. According to the illustrated embodiment, the first, or upper, two results, of descriptions 390, denoted as "unknown" in column 312, warrant further review of the corresponding episode data sets because the data sets could not be classified by the retrospective analysis, and the second two results, denoted as "SVT" in column 312, warrant further review because the retrospective analysis determined a different classification from that of the device analysis, which is shown in column 311 as "VT". According to alternate embodiments, page 30 includes descriptions of only those results warranting further review. It should be noted that a scrolling feature may be included on page 30 in order to view detailed information for a greater number of episodes on page 30.

Descriptions 390 associated with each episode in the detail report of page 30 also facilitate a more efficient review of the data associated with each episode. The following listing presents potential results descriptions that may be displayed in column 312:

| |
|---|
| Not Analyzed |
| Unknown |
| Ventricular Oversensing |
| SVT |
| Atrial Fib |
| Atrial Flutter |
| monomorphic VT |
| monomorphic VT |
| Atrial Undersensing |
| polyVT or VF |
| Atrial/Sinus Tach |
| Dual Tach |
| Induced |
| Induced |

The following listing presents potential evidence descriptions that may be displayed in column 313:

| |
|---|
| V morphology changed at onset of arrhythmia |
| V morphology matches a different episode that is most likely VT |
| More than 3 different V morphologies |
| V morphology changed at onset of arrhythmia |
| More than one V morphology |
| Consistent V morphology |
| ATP may terminate this type of monomorphic arrhythmia and reduce unnecessary shocks |
| More V than A events |
| AV association and Regular VV |
| More A than V events |
| More A than V events |
| VV slows after Rx but AA does not |
| Irregular VV |
| Same V morphology for considerably different V cycle lengths |
| Gradual onset and AV association |
| AA is regular when far-field R-waves are removed |
| AV association |
| AV event ratio is 1:1 |
| AA remains consistent even though Ventricular ATP is applied |
| Possibly VT with 1:1 retrograde conduction |
| Arrhythmia originated in the Atrium |
| Arrhythmia originated in the Ventricle |
| Could not determine which chamber initiated the arrhythmia |
| Excessively long AA intervals |
| Probable Atrial sensing issues |
| Could not validate Atrial sensing |
| Blocked Atrial beats observed |
| Fast Atrial rate |
| Could not determine AV event ratio |
| Possible oversensing of EMI (electromagnetic interference) |
| Possible oversensing of myopotentials |
| Possible double counting of R-waves |
| Possible T-wave oversensing |
| Possible oversensing due to lead failure |
| Possible double counting of R-waves |
| Possible T-wave oversensing |
| Possible oversensing due to lead failure |
| Possible oversensing due to loose set screw |
| Possible oversensing due to lead failure |
| Possible oversensing due to loose set screw |
| Possible oversensing on Ventricular egm |
| No lead impedance trend data stored by device. |
| Lead impedance trend data stored by device is not valid. |
| Lead impedance data suggests that leads were disconnected from device. |
| Lead impedance data for Gem VR devices with an interchangeable header is unreliable. |
| Lead impedance data was not analyzed because it was not stored by the device. |
| Lead impedance data was not analyzed because it is not valid. |
| Lead impedance data was not analyzed because it suggests that leads were disconnected from device. |
| Lead impedance data was not analyzed because it is unreliable for Gem VR devices with an interchangeable header. |
| VP lead impedance trend suggests insulation failure. |
| <leadType> lead impedance has a decreasing trend. This could be due to lead failure. |
| <leadType> lead impedance has three or more low values. This could be due to lead insulation failure. |
| <leadType> lead impedance has three or more high values. This could be due to lead conductor fracture. |
| <leadType> lead impedance has 3 or more high values. This could be due to a loose set screw. |
| <leadType> lead impedance has 3 or more high values. This could be due to |
| lead conductor fracture or loose set screw. |
| <leadType> lead impedance value is greater than the expected range. This could be due to lead conductor fracture. |
| <leadType> lead impedance value is less than the expected range. This could be due to lead insulation failure. |
| <leadType> lead has abnormal impedance and oversensing, which suggests lead failure. |
| Sensing integrity counter and non-sustained tachy episodes suggest significant Ventricular oversensing. This could be due to lead failure, loose set screw, oversensing of myopotentials, or double-counting of R-waves. |
| <nstCount attribute value> Non-sustained tachy episode(s). |
| Sensing integrity counter has identified <sicCount attribute value> short VV intervals. |
| Lead Impedance was outside expected range on <leadAlgorithmDate attribute value> |
| Outlier criteria met on <leadAlgorithmDate attribute value> |
| Slope Decrease detected on <leadAlgorithmDate attribute value> |
| Sensing integrity counter has estimated <sicPerDay attribute value> short VV intervals per day. |
| Episode classification not performed if no Atrial markers or intervals |
| Episode classification not performed for this episode type |
| Lead and Episode analysis not performed for this device/model. |
| Episode data is not valid |
| Episode classification not performed if no Ventricular egm channel stored |

Those users of the application, who are skilled in the art of electrophysiology and cardiac arrhythmias, will appreciate the information communicated by each description in the above listings, when displayed on page 30.

Figure 3 further illustrates columns 314-319 of page 30 including episode log information, which indicates whether or not device 10 terminated each episode (column 314), a date of each episode (column 315), a time associated with each episode (columns 316 and 317), an average heart rate associated with each episode (column 318), and an identification number associated with each episode (column 319). According to the illustrated embodiment, page 30 further includes a plurality of "view" interactive elements 354 for linking to the graphical display associated with each episode. Although Figure 3 illustrates one of elements 354 for each episode, according to alternate embodiments, element 354 is only displayed for those episodes for which the automated analysis provided results warranting further review of the associated data. When the user clicks on one of elements 354, a new page appears, which shows a graphical display of the data associated with the corresponding episode, for example page 40 shown in Figure 4A.

Figure 4A illustrates page 40 including a graphical display 45 of EGM strips and a corresponding marker channel, which is associated with an episode having identification number 15. Page 40 may be reached either by clicking on the element 354 associated with episode #15, which is displayed on page 30, or by clicking on "view episodes" element 254, which is displayed on page 20' (Figure 2B). Figure 4A further illustrates page 40 including a scroll box 41 to enable the user to select from the analyzed episodes, for example, by clicking on a desired episode within box 41; box 41 is particularly useful if the user has linked to page 40 by way of page 20'. Page 40 also displays an interactive feature 411 that allows the user to select the form of the graphical display.

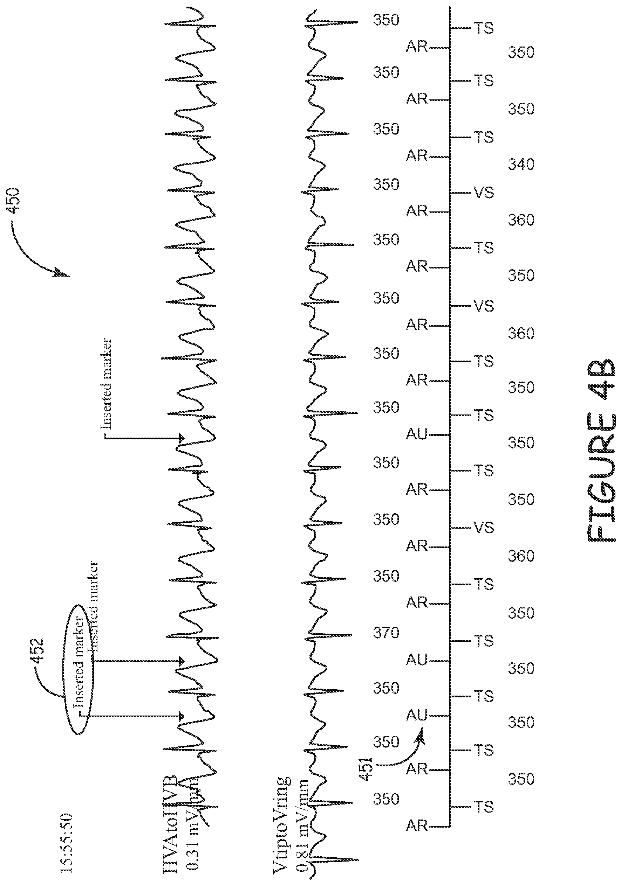

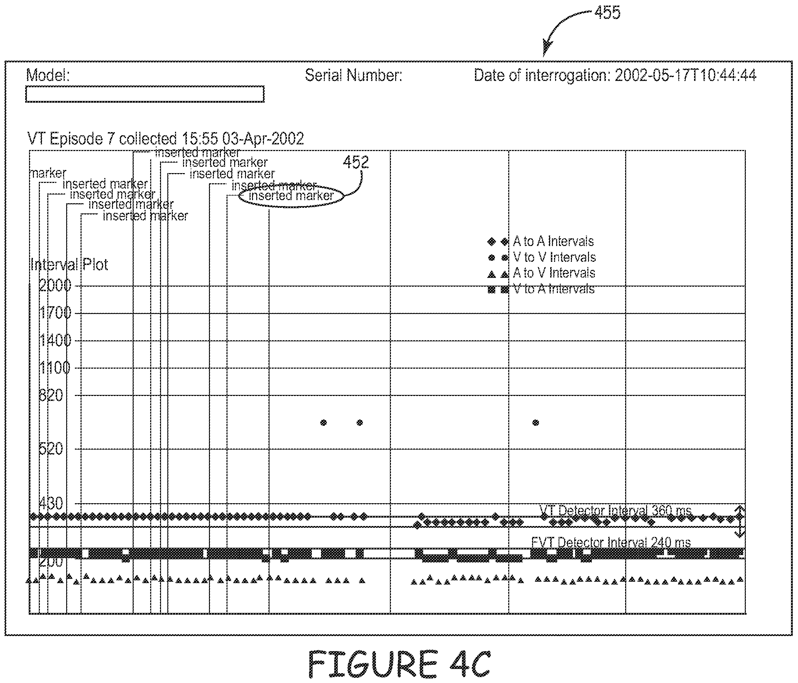

Figures 4B-C are examples of some forms of graphical displays, familiar to those skilled in the art, which may be presented on page 40. Figure 4B shows another display 450 of EGM strips and a corresponding marker channel, and Figure 4C shows an interval plot display 455; each of displays 450 and 455 have been corrected and annotated by software application 134 according to the analysis results for the corresponding data set, per the user's request on page 20/20' (Figures 2A-B). Figure 4B illustrates markers "AU" 451, which designate undersensed atrial events detected by the analysis, and "inserted marker" annotations 452 designating a location of each marker 451. Likewise Figure 4C illustrates inserted markers and associated "inserted marker" annotations 452. Returning now to Figure 2B, a user may click on interactive element 255 to view an impedance trend associated with report 29. Figure 5 is an example of an impedance trend plot 555 for atrial and ventricular pacing leads, for example leads 15, 17 of device 10 (Figure 1), according to some embodiments of the present invention. Plot 555, generated from data collected via periodic impedance measurements made by an implanted device, for example, device 10, provides a picture of how device impedance has changed over time in order to further facilitate an auxiliary analysis of device integrity results warranting further review.

An exemplary embodiment of an application user interface according to the invention includes a plurality of pages, the application being part of a system facilitating auxiliary analysis of data collected by an implanted medical device. The user interface further includes a first interactive element displayed on a first page of the plurality of pages, the first interactive element for selecting a group of data sets, the group of data sets included in a collection of data being stored in a data base and having been collected from the implanted device, and a second interactive element displayed on the first page, the second interactive element for invoking an automated analysis of each data set of the selected group of data sets, the automated analysis employing a pre-programmed algorithm to perform the analysis. The interface also includes a summary of results from the automated analysis displayed on the first page, after the automated analysis is invoked, the summary informing of each automated analysis result warranting further review of the corresponding data set; and a third interactive element displayed on the first page, after the automated analysis is invoked, the third interactive element for linking to a detailed report on a second page of the plurality of pages, the detailed report including a description of each automated analysis result warranting further review of the corresponding data set.

In one example, each of at least some of the selected data sets describe an episode detected and classified by an analysis performed by the implanted device; and the results warranting further review, that are associated with each episode-describing data set, differ in classification from a classification generated by the device analysis of the corresponding selected data set. A fourth interactive element displayed on the first page may be included, the fourth interactive element for enabling a view of a graphical display of each episode for which the automated analysis results warrant further review. The fourth interactive element, may be displayed on the first page, after the automated analysis is invoked, for linking to a third page of the plurality of pages on which a graphical display of each episode, for which the automated analysis results warrant further review, is viewable.

In another example, the user interface includes a fourth interactive element displayed on the first page, after the automated analysis is invoked, the fourth interactive element for linking to a third page of the plurality of pages on which the automated analysis results warranting further review are displayed in an XML format. In another example, the summary provides a warning if a device integrity issue is detected by the automated analysis, or the summary further provides evidence of a device integrity issue detected by the automated analysis.

In another example, the detailed report further includes a description of each automated analysis result that does not warrant further review of the corresponding data set; and the detailed report displays each automated analysis result warranting further review of the corresponding data set in a more prominent fashion than the results not warranting further review.

In another example, the detailed report further includes a description of each automated analysis result that does not warrant further review of the corresponding data set; and the detailed report displays the description of each automated analysis result warranting further review of the corresponding data set more boldly than the description of results not warranting further review. Or, the detailed report further includes a description of each automated analysis result that does not warrant further review of the corresponding data set; and the detailed report displays the description of each automated analysis result warranting further review of the corresponding data set in a different color from that of the description of results not warranting further review, or the detailed report displays the description of each automated analysis result warranting further review of the corresponding data set closer to a top of the page than the description of results not warranting further review. The description of each automated analysis result in the detailed report may include evidence supporting the result.

In another example, wherein the detailed report further includes episode log information for each episode for which the automated analysis results warrant further review, the episode log information including at least one of: a date of the corresponding episode, a time of the corresponding episode, an average heart rate of the corresponding episode, and an indication of outcome from therapy delivered by the implanted device in conjunction with the corresponding episode.

In yet another example, the interface also includes a plurality of interactive elements displayed on the second page in the detailed report, each interactive element of the plurality of interactive elements corresponding to the episode associated with each result warranting further review, and each of the plurality interactive elements linking to a third page on which a graphical display of the corresponding episode is viewable.

In conclusion, it may be appreciated that various embodiments of system described herein provide a tool to enable an automated analysis of data collected from implanted devices, and to present both options for the automated analysis and viewing of the analysis results in an organized and interactive fashion on a user interface. This tool can support, for a relatively large number of data sets, which may span a relatively large patient population, an efficient auxiliary analysis of implanted device data, on the part of clinicians and physicians. According to those embodiments of the present invention, wherein invocation of an analysis service is automated and the service collects data from a plurality of devices implanted in a plurality of patients at predetermined intervals, the analysis service, may provide a signal or notification, corresponding to those results warranting further review, for example, via a telephone call, an e-mail, a fax or page, to an attending physician or clinician, which notification indicates that one or more particular devices/patients require attention.

In the foregoing detailed description, the invention has been described with reference to specific embodiments. However, it may be appreciated that various modifications and changes can be made without departing from the scope of the invention as set forth in the appended claims.

## Claims

1. A system facilitating analysis of data collected by an implanted medical device, the system comprising:
a data handling system (120) for storing a collection of data, the collection of data including data sets collected by the implanted medical device, wherein at least a portion of the data sets comprises one or more episode-describing data sets that describe one or more episodes detected and classified by an analysis performed by the implanted medical device;
an analysis service (132) including a pre-programmed algorithm adapted to analyze and classify the episode-describing data sets; and
a software application (134) adapted to invoke the algorithm of the analysis service to analyze and classify a selected group of the data sets, organize results of the analysis service analysis for each of the selected group of data sets, and provide, via a user interface, a notification to a user of the system if any of the results are determined to be results warranting further review, **characterised in that** results for respective episode-describing data sets that have a classification generated by the analysis service analysis that differs from a classification generated by the analysis performed by the implanted medical device are determined to be results warranting further review; and
wherein the user interface includes a plurality of pages and further includes a first interactive element displayed on a first page of the plurality of pages and a second interactive element displayed on the first page; wherein
the software application is configured to cause the algorithm to select a group of data sets, in response to activation of the first interactive element by the user, the group of data sets included in the collection of data, and to invoke an automated analysis of each of the selected group of data sets in response to activation of the second interactive element by the user, using the algorithm of the analysis service; and to cause the user interface to display a summary of results from the analysis displayed on the first page, after the analysis is invoked, the summary indicating those results warranting further review by displaying these in a more prominent fashion than results not warranting further review; and the user interface including a third interactive element displayed on the first page, after the automated analysis is invoked, the software application configured to cause the algorithm to link to a detailed report on a second page of the plurality of pages in response to activation of the third interactive element by the user, the detailed report including a description of each result warranting further review.

2. The system of claim 1, wherein the described episode(s) detected is/are a type of cardiac arrhythmia episode.

3. The system of claim 1, wherein results indicative of a device integrity issue are determined to be results warranting further review.

4. The system of claim 1, wherein the preprogrammed algorithm is adapted to analyze each selected data set for an indication of one of supra-ventricular tachyarrhythmia, atrial fibrillation, atrial flutter, atrial/sinus tachyarrhythmia and monomorphic ventricular tachyarrhythmia.

5. The system of claim 1, wherein the software application provides, via the user interface, an interactive element to allow a user to invoke the analysis service through the software application.

6. The system of claim 1, wherein the software application invokes the algorithm of the analysis service automatically at periodic intervals.

7. The system of claim 1, wherein the report of the results is organized such that each result warranting further review is displayed bolder than results not warranting further review.

8. The system of claim 1, wherein the report of the results is organized such that each result warranting further review is displayed in a color different from a color in which results not warranting further review are displayed.

9. The system of claim 1, wherein the report of the results is organized such that each result warranting further review is located closer to a top of the display than results not warranting further review.

10. The system of claim 1, wherein the report further includes a description of evidence supporting each result warranting further review.

11. The system of claim 1, wherein the report further includes a time associated with the data set associated with each result warranting further review.

## Patentansprüche

1. System zum Erleichtern der Analyse von Daten, die von einer implantierten medizinischen Vorrichtung gesammelt werden, wobei das System Folgendes umfasst:
ein Datenverarbeitungssystem (120) zum Speichern einer Sammlung von Daten, wobei die Sammlung von Daten Datensätze beinhaltet, die von der implantierten medizinischen Vorrichtung gesammelt werden, wobei mindestens ein Teil der Datensätze einen oder mehrere Episoden-beschreibende Datensätze umfasst, die eine oder mehrere Episoden beschreiben, die von einer Analyse erkannt und eingestuft werden, die von der implantierten medizinischen Vorrichtung durchgeführt wird;
einen Analyseservice (132), der einen vorprogrammierten Algorithmus beinhaltet, der angepasst ist, um die Episodenbeschreibenden Datensätze zu analysieren und einzustufen; und
eine Softwareanwendung (134), die angepasst ist, um den Algorithmus des Analyseservice aufzurufen, um eine ausgewählte Gruppe der Datensätze zu analysieren und einzustufen, Ergebnisse der Analyse des Analyseservice für jede der ausgewählten Gruppe von Datensätzen zu organisieren und über eine Benutzerschnittstelle einem Benutzer des Systems eine Benachrichtigung bereitzustellen, wenn bestimmt wird, dass beliebige der Ergebnisse Ergebnisse sind, die eine weitere Überprüfung erfordern, **dadurch gekennzeichnet, dass** bestimmt wird, dass Ergebnisse für entsprechende Episoden-beschreibende Datensätze, die eine Einstufung aufweisen, die von der Analyse des Analyseservice generiert wird und von einer Einstufung abweicht, die von der Analyse generiert wird, die von der implantierten medizinischen Vorrichtung durchgeführt wird, Ergebnisse sind, die eine weitere Überprüfung erfordern; und
wobei die Benutzerschnittstelle eine Vielzahl von Seiten beinhaltet und ferner ein erstes interaktives Element, das auf einer ersten Seite der Vielzahl von Seiten angezeigt wird, und ein zweites interaktives Element, das auf der ersten Seite angezeigt wird, beinhaltet; wobei
die Softwareanwendung dazu konfiguriert ist, den Algorithmus dazu zu veranlassen, als Reaktion auf die Aktivierung des ersten interaktiven Elements durch den Benutzer eine Gruppe von Datensätze auszuwählen, wobei die Gruppe von Datensätzen in der Sammlung von Daten enthalten ist, und als Reaktion auf die Aktivierung des zweiten interaktiven Elements durch den Benutzer eine automatisierte Analyse von jeder der ausgewählten Gruppe von Datensätzen aufzurufen, wobei der Algorithmus des Analyseservice verwendet wird; und die Benutzerschnittstelle dazu zu veranlassen, eine Zusammenfassung der Ergebnisse aus der Analyse anzuzeigen, die auf der ersten Seite angezeigt wird, nachdem die Analyse aufgerufen wird, wobei die Zusammenfassung die Ergebnisse angibt, die eine weitere Überprüfung erfordern, indem diese in einer deutlicheren Weise als Ergebnisse, die keine weitere Überprüfung erfordern, angezeigt werden; und wobei die Benutzerschnittstelle ein drittes interaktives Element beinhaltet, das auf der ersten Seite angezeigt wird, nachdem die automatisierte Analyse aufgerufen wird, wobei die Softwareanwendung dazu konfiguriert ist, den Algorithmus dazu zu veranlassen, sich als Reaktion auf die Aktivierung des dritten interaktiven Elements durch den Benutzer mit einem ausführlichen Bericht auf einer zweiten Seite der Vielzahl von Seiten zu verknüpfen, wobei der ausführliche Bericht eine Beschreibung jedes Ergebnisses beinhaltet, das eine weitere Überprüfung erfordert.

2. System nach Anspruch 1, wobei das/die erkannte(n) beschriebene(n) Episode(n) einen Typ einer Herzrhythmusstörungsepisode aufweist/aufweisen.

3. System nach Anspruch 1, wobei bestimmt wird, dass Ergebnisse, die auf eine Vorrichtungsintegrität hinweisen, Ergebnisse sind, die eine weitere Überprüfung erfordern.

4. System nach Anspruch 1, wobei der vorprogrammierte Algorithmus angepasst ist, um jeden ausgewählten Datensatz für eine Angabe einer supraventrikulären Tachyarrhythmie, Vorhofflimmern, Vorhofflattern, Vorhof-/Sinus-Tachyarrhythmie und monomorphen ventrikulären Tachyarrhythmie zu analysieren.

5. System nach Anspruch 1, wobei die Softwareanwendung über die Benutzerschnittstelle ein interaktives Element bereitstellt, um einem Benutzer zu ermöglichen, den Analyseservice durch die Softwareanwendung aufzurufen.

6. System nach Anspruch 1, wobei die Softwareanwendung den Algorithmus des Analyseservice automatisch in regelmäßigen Abständen abruft.

7. System nach Anspruch 1, wobei der Bericht der Ergebnisse derart organisiert ist, dass jedes Ergebnis, das eine weitere Überprüfung erfordert, fetter angezeigt wird als Ergebnisse, die keine weitere Überprüfung erfordern.

8. System nach Anspruch 1, wobei der Bericht der Ergebnisse derart organisiert ist, dass jedes Ergebnis, das eine weitere Überprüfung erfordert, in einer Farbe angezeigt wird, die sich von einer Farbe unterscheidet, mit der Ergebnisse angezeigt werden, die keine weitere Überprüfung erfordern.

9. System nach Anspruch 1, wobei der Bericht der Ergebnisse derart organisiert ist, dass sich jedes Ergebnis, das eine weitere Überprüfung erfordert, näher an einem oberen Rand der Anzeige befindet als Ergebnisse, die keine weitere Überprüfung erfordern.

10. System nach Anspruch 1, wobei der Bericht ferner eine Beschreibung der Beweise beinhaltet, die jedes Ergebnis unterstützen, das eine weitere Überprüfung erfordert.

11. System nach Anspruch 1, wobei der Bericht ferner eine Zeit beinhaltet, die mit dem Datensatz verbunden ist, der mit jedem Ergebnis verbunden ist, das eine weitere Überprüfung erfordert.

## Revendications

1. Système facilitant l'analyse de données recueillies par un dispositif médical implanté, le système comprenant :
un système de traitement de données (120) permettant de stocker une collecte de données, la collecte de données comprenant des ensembles de données recueillies par le dispositif médical implanté, dans lequel au moins une partie des ensembles de données comprend un ou plusieurs ensembles de données décrivant des épisodes qui décrivent un ou plusieurs épisodes détectés et classés par une analyse effectuée par le dispositif médical implanté ;
un service d'analyse (132) comprenant un algorithme préprogrammé conçu pour analyser et classer les ensembles de données décrivant des épisodes ; et
une application logicielle (134) conçue pour appeler l'algorithme du service d'analyse pour analyser et classer un groupe sélectionné des ensembles de données, organiser les résultats de l'analyse du service d'analyse pour chaque groupe sélectionné d'ensembles de données, et fournir, via une interface utilisateur, une notification à un utilisateur du système si l'un des résultats est déterminé comme étant un résultat justifiant un examen plus approfondi, **caractérisé en ce que** les résultats pour les ensembles de données respectifs décrivant les épisodes qui ont une classification générée par l'analyse du service d'analyse qui diffère d'une classification générée par l'analyse effectuée par le dispositif médical implanté sont considérés comme des résultats justifiant un examen plus approfondi ; et dans lequel l'interface utilisateur comprend une pluralité de pages et comprend en outre un premier élément interactif affiché sur une première page de la pluralité de pages et un deuxième élément interactif affiché sur la première page ; dans lequel l'application logicielle est configurée pour amener l'algorithme à sélectionner un groupe d'ensembles de données, en réponse à l'activation du premier élément interactif par l'utilisateur, le groupe d'ensembles de données étant inclus dans la collecte de données, et à appeler une analyse automatisée pour chaque groupe d'ensembles de données sélectionné en réponse à l'activation du deuxième élément interactif par l'utilisateur, en utilisant l'algorithme du service d'analyse ; et à amener l'interface utilisateur à afficher un résumé des résultats issus de l'analyse affichés sur la première page, après la demande d'analyse, le résumé indiquant les résultats justifiant un examen plus approfondi en les affichant de façon plus visible que les résultats ne justifiant pas un examen plus approfondi; et l'interface utilisateur comprenant un troisième élément interactif affiché sur la première page, après l'appel de l'analyse automatisée, l'application logicielle étant configurée pour amener l'algorithme à établir un lien vers un rapport détaillé sur une seconde page de la pluralité de pages en réponse à l'activation du troisième élément interactif par l'utilisateur, le rapport détaillé comportant une description de chaque résultat justifiant un examen plus approfondi.

2. Système selon la revendication 1, dans lequel l'épisode ou les épisodes décrit(s) détecté(s) est/sont un type d'épisode d'arythmie cardiaque.

3. Système selon la revendication 1, dans lequel les résultats indiquant un problème d'intégrité de dispositif sont déterminés comme étant des résultats justifiant un examen plus approfondi.

4. Système selon la revendication 1, dans lequel l'algorithme préprogrammé est conçu pour analyser chaque ensemble de données sélectionné pour indiquer l'un des éléments suivants : une tachyarythmie supra-ventriculaire, une fibrillation auriculaire, un flutter auriculaire, une tachyarythmie auriculaire/sinusale et une tachyarythmie ventriculaire monomorphe.

5. Système selon la revendication 1, dans lequel l'application logicielle fournit, via l'interface utilisateur, un élément interactif pour permettre à un utilisateur d'appeler le service d'analyse à travers l'application logicielle.

6. Système selon la revendication 1, dans lequel l'application logicielle appelle automatiquement l'algorithme du service d'analyse à intervalles périodiques.

7. Système selon la revendication 1, dans lequel le rapport des résultats est organisé de sorte que chaque résultat justifiant un examen plus approfondi soit affiché en caractère plus gras que les résultats ne justifiant pas un examen plus approfondi.

8. Système selon la revendication 1, dans lequel le rapport des résultats est organisé de sorte que chaque résultat justifiant un examen plus approfondi soit affiché dans une couleur différente d'une couleur dans laquelle sont affichés les résultats ne justifiant pas un examen plus approfondi.

9. Système selon la revendication 1, dans lequel le rapport des résultats est organisé de sorte que chaque résultat justifiant un examen plus approfondi soit situé plus près de la partie supérieure de l'écran que les résultats ne justifiant pas un examen plus approfondi.

10. Système selon la revendication 1, dans lequel le rapport comprend en outre une description des preuves à l'appui de chaque résultat justifiant un examen plus approfondi.

11. Système selon la revendication 1, dans lequel le rapport comprend en outre un temps associé à l'ensemble de données associé à chaque résultat justifiant un examen plus approfondi.
